# EUROPEAN PATENT SPECIFICATION

(11) **EP 4 090 295 B1**
(45) Date of publication and mention of the grant of the patent: **11.11.2026**
(21) Application number: 20766664.5
(22) Date of filing: 04.03.2020
(51) Int. Cl.: A61F 2/58, B25J 9/00, B25J 9/10, B25J 13/00

(54) **VOICE-ACTIVATED, COMPACT, AND PORTABLE ROBOTIC SYSTEM**
SPRACHAKTIVIERTES, KOMPAKTES UND TRAGBARES ROBOTERSYSTEM
SYSTÈME ROBOTIQUE À COMMANDE VOCALE, COMPACT ET PORTATIF

(30) Priority: 04.03.2019 US 201962813200 P
(43) Date of publication of application: 23.11.2022
(73) Proprietor: Georgia Tech Research Corporation, Atlanta, GA 30318 (US)
(72) Inventor: DESAI, Jaydev, Atlanta, Georgia 30318 (US); JEONG, Seokhwan, Atlanta, Georgia 30318 (US); TRAN, Phillip, Atlanta, Georgia 30318 (US); WANG, Xuefeng, Atlanta, Georgia 30318 (US)
(74) Representative: Wittmann, Günther
(86) International application number: PCT/US2020/020954
(87) International publication number: WO 2020/180965

(56) References cited:
- WO-A1-2017/072463
- WO-A1-2017/072463
- GB-A- 2 552 358
- KR-Y1- 200 349 089
- US-A1- 2006 094 989
- US-A1- 2006 094 989
- US-A1- 2006 282 936
- US-A1- 2006 282 936
- US-A1- 2013 096 477
- US-A1- 2014 142 470
- US-A1- 2017 168 565
- US-A1- 2017 168 565
- US-B1- 6 427 248

## Description

### BACKGROUND OF THE INVENTION

### 1. Field of the Invention

The present invention relates to a exoskeleton system .

### 2. Description of the Related Art

Spinal cord injury (SCI) is a neurological disorder that results in a variety of disabilities, examples of which include spasticity, cardiac dysfunction, and complete loss of motor function and control of hand movement. The inability of SCI patients to voluntarily move their hands decreases both their quality of life and their independence. Stroke patients have similar difficulties. Thus, much effort has been dedicated to realizing methods for improving and/or restoring hand function in those with impaired hand function.

Physical therapy and rehabilitation through functional hand exercises in the form of activities of daily living can facilitate the recovery of hand function in people with degraded or nonexistent motor control, such as SCI and stroke patients. Since the ability of the patient to perform hand movements independently can be compromised, assistance must be provided to the patient to successfully accomplish rehabilitation tasks. Traditionally, such assistance is in the form of a therapist moving a patient's hand and fingers to exercise the muscles. However, such therapy requires direct therapist involvement with a single patient during an entire therapy session.
fingers to exercise the muscles. However, such therapy requires direct therapist involvement with a single patient during an entire therapy session.

Several robotic rehabilitation devices have been developed to assist both patients and therapists during rehabilitation. Also, permanent assistive devices for carrying out activities of daily living (ADL) have been developed. However, development of such robotic devices is still in an early stage. One type of such a device is a robotic rehabilitation exoskeleton that is attached to a patient's hand and that causes a predetermined pattern of movement in the hand.

Rehabilitation exoskeletons can generally be classified into two broad categories based on the design and actuation technique: traditional rigid kinematic structures and soft robotic systems. Traditional exoskeletons typically feature a rigid construction and utilize gears, linkages, and pulleys that are directly integrated into the exoskeleton itself to drive actuation. A disadvantage of these devices is that movement restriction is caused by bulky designs and increased exoskeleton weight.

One potential solution to this disadvantage is through soft robotics. Soft robotic exoskeletons conform to the shape of the hand and use a minimal number of moving components. This reduces the size, weight, and complexity of the design and can improve range-of-motion (ROM) due to the decreased number of restrictive elements placed on the hand. Various methods of actuation are used in soft exoskeletons, such as tendon-drive, pneumatic, and hydraulic systems. However, current actuation designs tend not to be portable and tend to restrict overall ROM of the patient. Pneumatic-driven and hydraulic-driven systems require relatively large and noisy pumps to function properly. In tendon-driven systems, the main design obstacle is the implementation of bi-directional actuation for flexion and extension of the fingers. Current tendon-drive designs utilize pulley systems with a large footprint, thus necessitating an immobile actuation block.

To enable exoskeleton control, rehabilitation exoskeletons utilize many different methods such as electromyography (EMG), vision-based classification, and manual control by another person. A common disadvantage among these control strategies is the requirement of a static computing setup connected to the exoskeleton, thus restricting the mobility of the user and limiting the ROM of the user's upper body. Other important aspects include the user-friendliness, intuition, and implementation required for exoskeleton control. EMG-based control may be difficult to implement in SCI patients as a result of required nerve connections having been severed, thus requiring biosensor inputs from nerves not directly connected to the hand, such as the shoulder. EMG is also susceptible to external noise and signal recognition of EMG can differ between SCI patients. Manual control does not require much setup but it is not intuitive because there may be a disconnect between user intent and device actuation wherein the user is indirectly controlling the exoskeleton through another person.

WO 2017/072463 A1 discloses a motorized for a hand, comprising: at least one first articulated digital structure for surrounding the phalanges of a first finger and having an inner part and an outer part; a motor; a first cable mounted slidably on the inner part of the first digital structure and having an end connected to the motor, in such a way that the actuation of the motor exerts tension on the first cable in order to cause the first finger to move to a flexed position. The exoskeleton has a first elastic means mounted slidably on the outer part of the digital structure, in such a way that the first finger automatically adopts an extended position when the motor does not exert tension on the first cable.

US 2017/0168565 A1 provides a wearable device including at least one compliant region adapted and configured to be placed over a joint of a subject and at least two flexible but less compliant regions coupled to opposite ends of the compliant region. The wearable device comprises a wearable robotic device including a wearable device as described herein and at least one actuator adapted and configured to move the flexible but less compliant regions relative to each other.

Therefore, there is a need for a robotic exoskeleton system that has a minimal impact on range of motion and that can operate without continuous direct therapist involvement.

### SUMMARY OF THE INVENTION

The object of the present invention is solved by the exoskeleton system according to claim 1.

### BRIEF DESCRIPTION OF THE FIGURES OF THE DRAWINGS

FIGS. 1A - **1B** are schematic diagrams showing one representative embodiment of a joint movement device for a joint with two joint members.
FIGS. 2A - 2B are schematic diagrams showing one representative embodiment of a joint movement device for a joint with three joint members.
FIG. 3 is a schematic diagram showing one representative embodiment of an actuator.
FIGS. 4A - 4B are schematic diagrams showing an example of an actuator not falling under the scope of the claims.
**FIG. 5** is a schematic diagram showing an embodiment employing a spring-type elastic member.
**FIG. 6** is a schematic diagram showing a joint movement system employing voice activation.
**FIGS. 7A - 7B** are schematic diagrams showing a palm and a back of a hand wearing a robotic exoskeleton system.
**FIG. 8** is a flow diagram demonstrating a method of actuating an exoskeleton using voice commands.
**FIGS. 9A - 9B** are a palm view and a back-hand view of an experimental embodiment of an exoskeleton system applied to a human hand.
**FIG. 10** is a schematic diagram showing an embodiment employing suction cups.
**FIGS. 11A - 11B** are views of an exoskeleton employing suction cups applied to a human hand.

### DETAILED DESCRIPTION OF THE INVENTION

A preferred embodiment of the invention is now described in detail. Referring to the drawings, like numbers indicate like parts throughout the views. Unless otherwise specifically indicated in the disclosure that follows, the drawings are not necessarily drawn to scale. The present disclosure should in no way be limited to the exemplary implementations and techniques illustrated in the drawings and described below. As used in the description herein and throughout the claims, the following terms take the meanings explicitly associated herein, unless the context clearly dictates otherwise: the meaning of "a," "an," and "the" includes plural reference, the meaning of "in" includes "in" and "on."

To overcome limitations of existing systems, one embodiment of the invention employs a tendon-actuated, voice-controlled soft robotic exoskeleton with 4 degrees-of-freedom for improving hand function in SCI patients. The actuation mechanism utilizes both active and passive actuation techniques to create a compact and lightweight system that can be carried on the forearm to improve patient mobility. Additionally, the use of voice control through a smartphone app removes the need for bulky control units, further increasing the portability of the exoskeleton and providing an intuitive control interface.

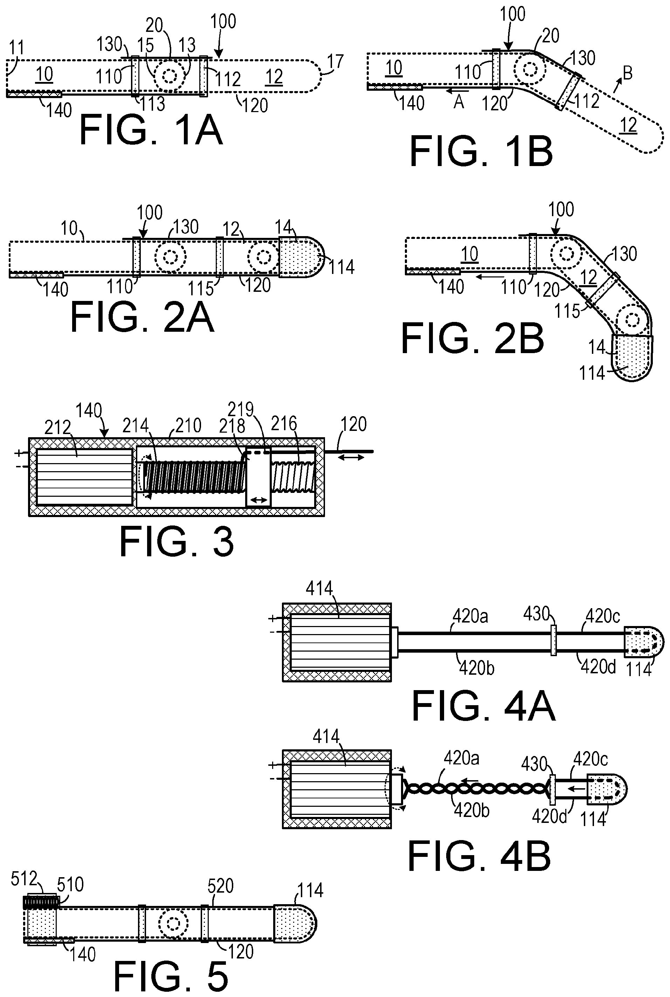

As shown in FIGS. 1A and 1B, one representative embodiment of the invention is a joint movement device **100** for selective flexion and extension of a joint **20** having a first joint member **10** having a proximal end **11** and an opposite distal end **13** and a second joint member **12** having a proximal end **15** and an opposite distal end **17** and the joint coupling the distal end **13** of the first joint member **10** to the proximal end **15** of the second joint member **12** so that the second joint member **12** is in a flexural relationship to the first joint member **10.**

A first tendon **120** is disposed adjacent to a selected side of the first joint member **10** and the second joint member **12.** When the first joint member **10** and the second joint member **12** are parts of a finger, the selected side can be either the palmar side or the dorsal side of the finger.

A first phalange ring **110** is secured to the first joint member **10** and a tendon securing device **112** (which could have the configuration of a second phalange ring, or a thimble-like cap) is secured to the second joint member **12.** The phalange ring **110** and tendon securing device **112** each have a tending routing mechanism **113** configured to route the tendon from the tendon securing device **114** through the phalange ring **110.** In one embodiment, the routing mechanism **113** includes a passage that is lined with a poly(tetrafluoroethylene) (e.g., Teflon^{®}) liner so as to minimize friction on the tendon **120.** (Also, other tubing used to route and house portions of the tendon **120** can include poly(tetrafluoroethylene) to minimize friction.)

An actuator **140** is placed in a fixed relationship with the first joint member **10** and is coupled to the tendon **120.** As shown in FIG. 1B, the actuator **140** acts as a shortening mechanism that shortens (as in the direction of arrow **A**) a portion of the first tendon **120** between the first phalange ring **110** and the tendon securing device **112,** thereby causing the joint **20** to flex. An elastic member, such as a nickel titanium alloy (such as Nitinol) wire **130,** is coupled to the first phalange ring **110** and the tendon securing device **114** so as to apply an extension force (as in the direction of arrow **B**), which causes the joint **20** to extend when the actuator **140** releases the tendon.

In certain embodiments, as shown in FIGS. 2A and 2B, the joint movement device **100** can be adapted for use with a finger-type joint that includes a third joint member **14.** In such an embodiment, a thimble-like cap **114** can act as a tendon securing device while a second phalange ring **115** can be used to route the tendon **120...**

According to the invention, as shown in FIG. 3, the actuator **140** includes a housing **210** that holds an electric motor **212** therein. A threaded member **214** is rotated by the motor **212** and includes a continuous spiral root **216** having a pitch corresponding to the dimeter of the tendon **120.** A nut **218** is threaded onto the threaded member **214** and is held by the housing **210** so that when the motor **212** rotates the threaded member **214,** the nut **218** moves laterally along the threaded member **214** in a direction corresponding to the direction of rotation. The tendon **120** runs through a passage **218** defined by the nut **218** and is wound around the threaded member **214** as it rotates in a first direction. This pulls the tendon **120** into the housing **210** and the tendon **120** is wrapped around the root **216** of the threaded member **214.** When the threaded member **214** rotates in the opposite direction, the tendon **120** is unwrapped from the root **216** and a portion of it is released from the housing **210.**

In an example not falling under the scope of the claims, as shown in FIGS. 4A and 4B, the tendon includes first portion **420a** and a second portion **420b,** which are attached to the spindle of a motor **414** and which are separated by a tendon separator **430,** a third portion **420c** and a fourth portion **420d,** which are attached to the cap **114.** As the motor **414** causes the spindle to spin in a first direction, the first portion **420a** and the second portion **420b** twist about each other, which causes the third portion **420c** and the fourth portion **420d** to draw in and effectively shorten. Spindle rotation in the opposite direction releases the tendon for joint extension.

In one alternative embodiment, as shown in FIG. 5, the elastic member can include a spring **510** that is affixed to the joint via a strap **512** and that is coupled to a cord **520** that is coupled to the cap **114** and that is disposed on a side of the joint opposite from the tendon **120.** The spring maintains the cord **520** under tension for joint extension when the actuator **140** releases the tendon **120.**

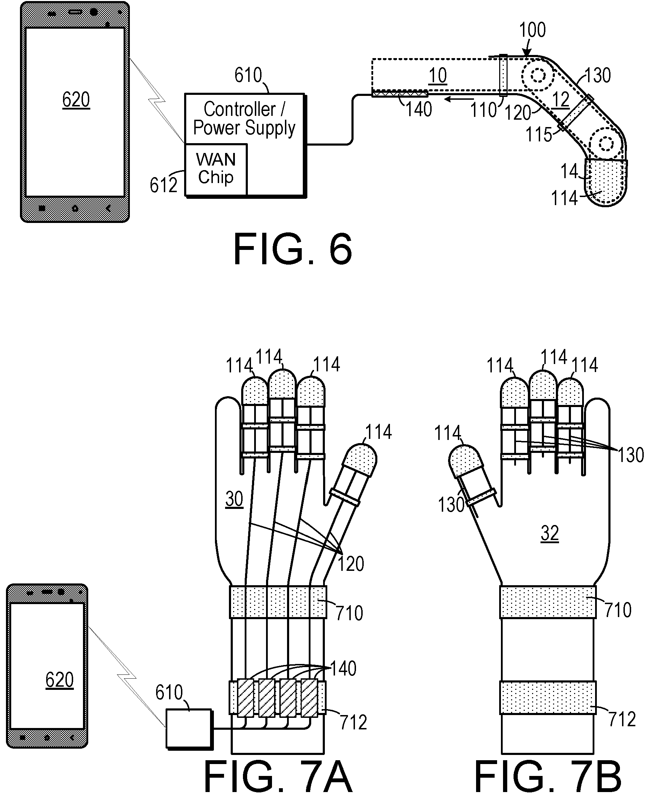

As shown in FIG. 6, a controller **610** applies control voltages to the actuator **140.** The controller **610** can include a wireless chipset **612** (e.g., a Bluetooth^{®} chipset) to transmit voice and other commands from a wireless device **620** (e.g., a smart phone, a tablet, a laptop, a dedicated device, etc.) to the actuator **140.** This allows a user to speak commands regarding the flexing of the joint into the wireless device **620** and the controller **610** then generates control voltages to the actuator. For example, if a plurality of joint movement devices **100** are used as a patient's hand robotic exoskeleton, then the patient can speak voice commands into the wireless device **620** to effect a hand exercise routine.

Such a hand exercise robotic exoskeleton system is shown in FIGS. 7A and 7B, which shows a view of the palm **30** and the back **32** of the patient's hand. This embodiment employs a routing strap **710** to route the tendons **120** and an actuator strap **712** to hold the actuators **140** in a fixed relationship with the patient's hand. The components of the exoskeleton system can be embedded in a glove for easy application to the patient.

In this embodiment, the wireless device can be programmed to effect predetermined actions in response to specific voice commands. Such commands could require the fingers to move in a certain way, such as: pinching, grabbing or grasping an object, or to open. Different types of objects can be specified. For example, the types of objects could include: a ball, a pen, a block, a coin or a checker. A certain type of movement applied to a certain object will result in a specific movement by the fingers. The voice commands may also include a command to "Stop Listening" and a command to "Resume Listening." The "Stop Listening" command will prevent spurious movements due to background conversations.

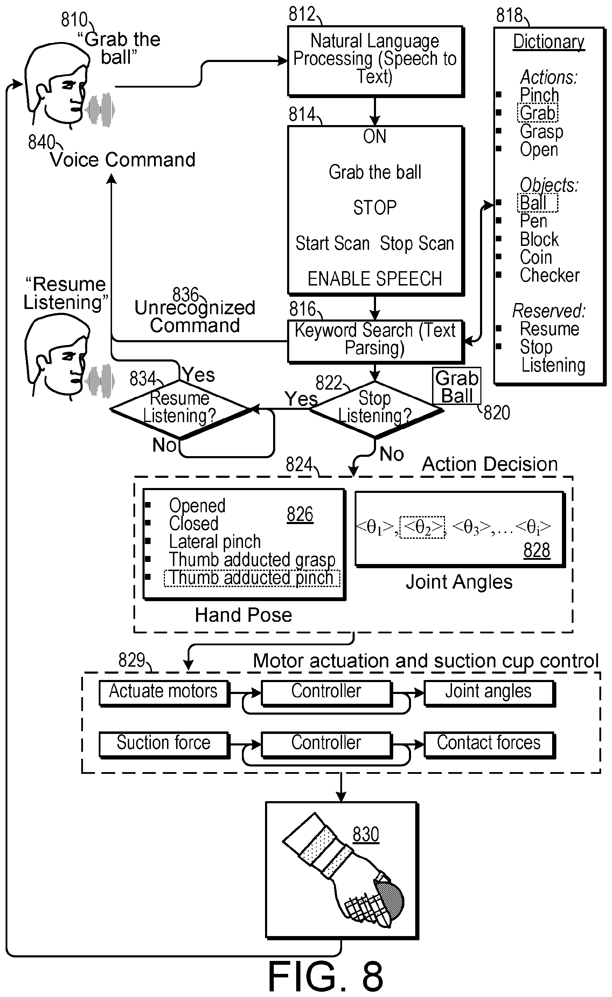

In one example, as shown in FIG. 8, the user says "Grab the ball" **810** which causes the wireless device first to transform the speech input to corresponding text **812.** The command is deciphered **814** and keywords from the command are parsed **816** by accessing a dictionary **818.** Once the command is understood, the system begins a "Grab Ball" routine **820** and makes an action decision **824** that includes taking predetermined actions directing the exoskeleton to move into a specific hand pose **826** with the joints at predetermined joint angles **828.** Motor actuation and suction cup control **829** apply the actuation force to the controller to achieve the desired joint angles and apply desired suction forces to the controller to achieve the desired level of contact force (which will be described in more detail below), respectively. Next, the commands are transformed to control voltages to the actuators that cause the exoskeleton **830** to move accordingly. Once the action is complete, the system returns to a state in which it awaits another command **840.** If the result of the Keyword Search is that the command is not recognized **836,** the system will return to the state in which it awaits another command **840.** This will also happen if the system has been put in a "Stop Listening" state **822** and has been commanded to "Resume Listening" **834.**

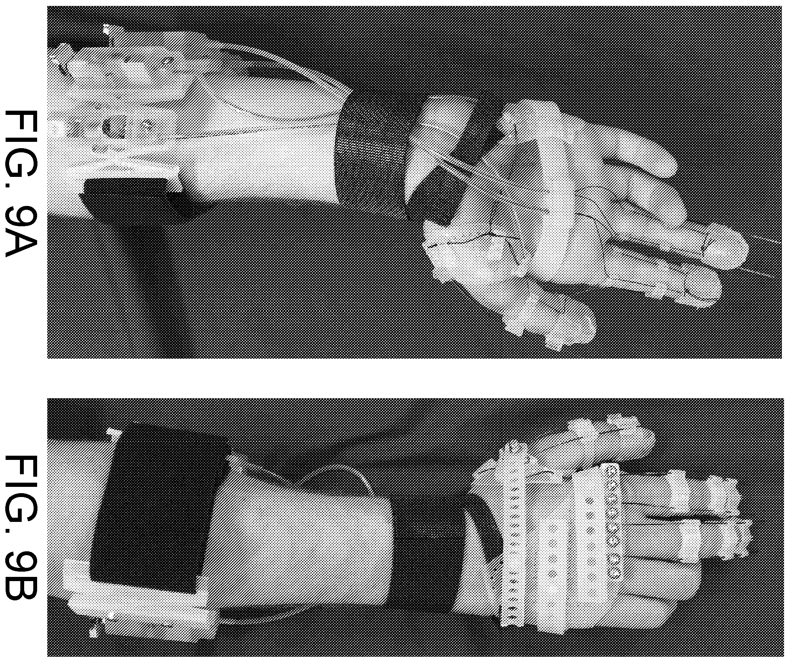

Extrinsic tendons located in the forearm are the main contributors for generating finger motion in the human hand. One experimental embodiment is shown in FIGS. 9A and 9B, which shows the palm side of an exoskeleton in FIG. 9A and a back hand side in FIG. 9B. This experimental embodiment utilizes a partially biomimetic tendon routing strategy through underactuation to achieve a simple yet effective finger flexion mechanism.

In the human hand, the flexor digitorum profundus tendons are responsible for the concurrent flexion of the metacarpophalangeal (MCP), proximal interphalangeal (PIP), and distal interphalangeal (DIP) joints in the index finger and the middle finger. This action is mimicked by the flexor tendon pairs in the experimental embodiment. The exotendons are organized in pairs to prevent force imbalance and improve stability during actuation.

The human thumb can be treated as a multi-degree of freedom (DoF) actuator that can perform many complex movements. This is mainly due to the carpometacarpal (CMC) joint, which allows for abduction and adduction to create opposable thumb movement. The adduction movement can be generated by a single tendon. The thumb is also capable of flexion through the action of the flexor pollicis longus tendon. This tendon is replicated by the thumb flexor tendon, which passes across the MCP and interphalangeal (IP) joints. Force transmission may be difficult to evenly distribute for CMC movement because of the non-uniform shape of the thenar eminence of the thumb.

To prevent attachment point shift, the adduction tendon in the experimental embodiment is wrapped around the thenar eminence. Thus, when actuated, the exotendon will tighten around the thenar eminence to distribute the contact force for improved stability.

The tendon-driven actuation used in the exoskeleton system can generate only unidirectional actuation. To implement finger flexion and extension, either active or passive actuation can be utilized. For active extension and flexion, each DoF would require two actuators to control both extension and flexion. This necessitates precise coordination of simultaneous tendon actuation to avoid interference between the extension and flexion movements. Thus, to avoid increasing the number of actuators and to decrease system complexity, passive actuation can be used for finger extension. In passive actuation, mechanical components such as a spring can be connected to the extension tendon in series and provide pretension to support extension. However, this can cause discomfort. Therefore, the passive component can be connected to the finger in parallel, which provides a force perpendicular to the finger without stress on the finger joints.

One experimental embodiment employs a passive backbone with nickel titanium alloy wires. The wires can be placed along the dorsal aspect of the index finger, middle finger, and thumb and are housed in the phalange rings. The nickel titanium alloy wires are superelastic and can withstand large strain. Therefore, the wires can withstand the high curvature bending caused by flexion of each joint without permanent deformation. The nickel titanium alloy wires mimic the function of the extensor digitorum communis tendon in the index finger and middle finger and the extensor pollicis longus tendon in the thumb to provide a compact flexion mechanism. As the actuator releases tendon tension, the nickel titanium alloy wire backbones exert torque on the finger joints to extend the finger to a predetermined resting posture.

Additionally, the backbone actuation mechanism employing superelastic wires inherently removes the problem of hyperextension. The backbone will return to its initial position upon tendon release by the actuator and so long as the fingers are not placed in a hyper-extended posture during device setup, the backbone will not over-rotate the joints. Also, this device can be used as a rehabilitative/assistive device for stroke patients. However, since stroke patients often have inherently closed hands, the tendons can run along the back sides of their hands (essentially, the device is flipped back-to-front) so that the tendons will pull the fingers into an open position.

The primary wearable components of the experimental embodiment (as shown in FIGS. 9A and 9B) can be fabricated using a deformable, low-friction 3D- printed plastic (such as, Durable Resin, available from FormLabs) and can be customized to the specifications of each user. The primary components consist of the phalange rings, CMC sleeve, and palm shell. Due to the deformability of the plastic, the phalange rings are able to be sized slightly smaller than the user's fingers to ensure a secure fit. Each ring routes the exotendons (which, in the experimental embodiment, were made from Hercules PE Braided Spectra, available from HerculesPro.com) along the sides of the finger to reduce obstruction of the palmar aspect of the finger, which can improve grasping and tactile sensation. The rings route the nickel titanium alloy wires slightly offset from the dorsal aspect of the fingers to prevent direct contact between the wires and the fingers during motion. The cap is thimble-shaped to wrap around the entire fingertip and made of 3D-printed plastic with a higher surface friction coefficient (such as, Elastic Resin, available from FormLabs, Inc.) on the palmar aspect of the fingertip to improve grip. The CMC sleeve is 3D-printed in a single piece and fits around the thenar eminence of the thumb. The adductor tendon routing strategy takes advantage of the structure of the CMC sleeve to provide force distribution around the thenar eminence during actuation to increase motion stability. The CMC sleeve decouples the flexion/extension motion of the thumb from adduction/abduction by directly connecting the thumb flexion tendon to the sleeve as well as anchoring the thumb extension nickel titanium alloy wires to the sleeve itself. Thus, flexion and extension of the thumb occurs relative to the CMC movement to prevent interference between the two DoFs of the thumb.

The secondary wearable components of the exoskeleton are the nickel titanium alloy wires and silicone bands (such as, Dragon Skin 30, available from Smooth-On, Inc.). The nickel titanium alloy wires are placed along the dorsal aspect of the fingers and are anchored to the palm shell close to the MCP joints at the dorsal surface of the hand. The nickel titanium alloy wires are placed along both sides of the finger to improve stability during motion and to help keep the phalange rings and the cap aligned on the finger. Two silicone bands are used in the one experimental embodiment. One band is used for passive abduction of the CMC and another band is used to secure the palm shell to the hand. The palm silicone band contains two insertion points to route the flexion tendon pairs of the index finger and middle finger down the center of the palm to the actuators. During human subject testing, it was observed that a rigid support structure placed across the palm can cause tissue to accumulate on the palm under the MCP joint of the finger during flexion, which impedes MCP joint displacement. The palm silicone band can deform with the palm during finger motion and thus, avoids tissue buildup and does not restrict the motion of the MCP joint.

To satisfy the requirements of an intuitive and user-friendly interface as well as system portability, the experimental embodiment uses discrete, task-based voice control for device operation. The voice control employed by the experimental embodiment was made compact by requiring only a smartphone (such as Galaxy S8, available from Samsung Electronics Co., Ltd) for onboard signal processing and a Bluetooth Low Energy (BLE) capable microcontroller (SAM3X8E, available from Microchip Technology Inc.) for controlling the actuators.

The app employed with the experimental embodiment used continuous voice control through continuous speech recognition. This allows the experimental embodiment to operate as a hands-free device so that the user does not need to divide their attention between the rehabilitation task and the smartphone.

Since a discrete, task-based controller was used, the user can create a customized dictionary to define exoskeleton actuation for different actions. For example, the user can identify the "cup" and "mug" objects as well as the "Grab" action. Then, different hand configurations can be assigned to "Grab the cup" and "Grab the mug." Thus, commands containing different keywords will actuate the fingers uniquely

The joint angles of the fingers for different actions are preset through the use of motor encoders. To begin device operation, the user gives a command to the app. The command is deciphered through natural language processing and parsed for keywords in the dictionary to identify the intended action. When a recognized command is detected, the relevant course of action is started by sending a unique identifier corresponding to the command from the app to the microcontroller via BLE. Alternately, since the experimental embodiment uses continuous voice control, the user can tell the app to "Stop Listening," wherein the app will ignore all further commands until the user says "Resume Listening" to continue exoskeleton control. The keywords determine the joint angles and hand pose associated with the action to be performed. For example, grabbing a block requires a specific set of joint angles for the index finger and the thumb as well as the "Thumb adducted pinch" hand pose. By separating actions into the constituent joint angles and hand pose, the experimental embodiment can classify different actions as requested by the user, simplifying the process of creating user-specific commands for the exoskeleton.

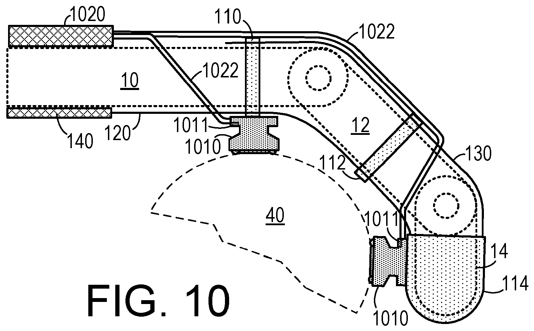

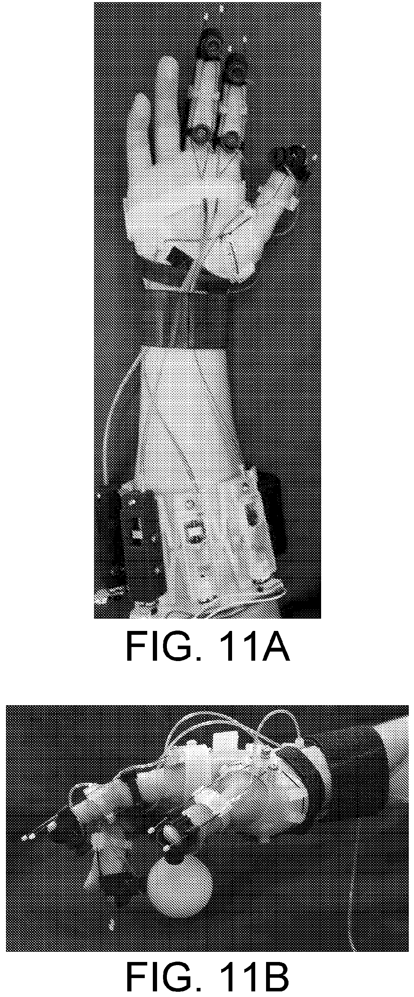

In certain embodiments, the caps 114 can be given tactile surfaces to facilitate grabbing. Also, as shown in FIGS. 10 and 11A-11B, controllable suction cups **1010** can be affixed to the caps 114 and other parts of the system. Such suction cups **1010** can be controlled so that decreased amounts of suction are applied as the user builds up grabbing strength. Suction in the suction cups **1010** can be controlled by a suction controller **1020** that maintains suction at a predetermined amount by pumping air from the suction cups **1010** through pneumatic tubes **1022.** Force sensors may be embedded in the base of the suction cups **1010** to provide real time feedback to the suction controller **1020** so as to maintain a desired level of suction. This view shows the suction cups **1010** assisting in gripping an object **40.** If the device is used as a rehabilitation device then the suction pressure can be decreased over time as the subject gets better during the rehabilitation regimen. Alternatively, if the device is used as an assist device then the suction will be engaged all the time when needed. Hence, through the voice command and the task requirement, like lifting an iron object versus an egg will lead to varying magnitudes of suction force to lift/manipulate the object. Hence, based on the voice command given the system should adapt the suction pressure. One example of a suction cup-type gripper is disclosed in US Patent 8,382,174. One embodiment employs sensors **1011** (including pressure sensors, force sensors and other types of sensors, depending on the specific application) at the base of the suction cups for measuring the grasping forces in real-time to control the amount of suction force that is exerted.

## Claims

1. An exoskeleton system configured for use with a plurality of different fingers of a hand and comprising a plurality of joint movement devices (100),
wherein the joint movement device (100) is adapted for selective flexion and extension of a joint (20) having a first joint member (10) having a proximal end (11) and an opposite distal end (13) and a second joint member (12) having a proximal end (15) and an opposite distal end (17) and the joint coupling the distal end (13) of the first joint member (10) to the proximal end (15) of the second joint member (12) so that the second joint member (12) is in a flexural relationship to the first joint member (10),
wherein each of the plurality of joint movement devices (100) is secured to a different corresponding finger of the hand, so that each joint movement device (100) is configured to cause the corresponding finger to move in a predetermined manner,
wherein the joint movement device (100) comprises:
(a) a tendon (120), having a first end and an opposite second end, disposed adjacent to a selected side of the first joint member (10) and the second joint member (12);
(b) a tendon securing device (112) secured to the distal end (17) of the second joint member (12) to which the tendon (120) is secured;
(c) at least one phalange ring (110) secured to the first joint member (10), the at least one phalange ring (110) and the tendon securing device (112) including a tending routing mechanism (113) configured to route the tendon (120) from the tendon securing device (112) through the at least one phalange ring (110);
(d) an actuator (140) in a fixed relationship with the first joint member (10) and coupled to the tendon (120), the actuator (140) configured to selectively pull the tendon (120) inwardly thereto, thereby causing the joint (20) to flex; and
(e) an elastic member (130) coupled to both the phalange ring (110) and the tendon securing device (112) and configured to apply an extension force to the tendon securing device (112), thereby causing the joint (20) to extend when the actuator (140) releases the tendon;
wherein the actuator (140) comprises:
(f) a tendon shortening mechanism configured to shorten a portion of the first tendon (120) between the phalange ring (110) and the tendon securing device (112); and
(g) a controller (610) configured to activate the tendon shortening mechanism, wherein the tendon shortening mechanism comprises:
(h) a housing (210);
(i) an electric motor (212) coupled to the housing (210);
(j) a threaded member (214) coupled to the motor (212) so as to be rotatable thereby; and
(k) a nut (218) in threaded relationship with the threaded member (214), the nut (218) held by the housing (210) so that when the motor (212) rotates the threaded member (214) , the nut (218) moves laterally along the threaded member (214), the nut (218) defining a passage (218) through which the tendon (120) passes,
**characterized in that**
(l) the threaded member (214) including a continuous spiral root (216) having a pitch corresponding to a diameter of the tendon (120) wherein the second end of the tendon (120) is secured to the threaded member (214) so that when the threaded member (214) rotates in a first direction, the tendon (120) is pulled into the housing (210) and wrapped around the continuous spiral root (216) and so that when the threaded member (214) rotates in a second direction, opposite the first direction, the tendon (120) is unwrapped from the continuous spiral root (216) and released from the housing (210).

2. The exoskeleton system of Claim 1, wherein the tending routing mechanism (113) of each phalange ring (110) includes a passage defined through which the tendon (120) passes that is lined with a poly(tetrafluoroethylene) liner so as to reduce friction on the tendon (120).

3. The exoskeleton system, wherein the elastic member (130) comprises a spring (510) having one end in a fixed relationship with the first joint (20) and a second end coupled to a cord (520) that is coupled to the cap (114) and that is disposed on a side of the joint (20) opposite from the tendon (120).

4. The exoskeleton system of Claim 1, wherein the controller is in control communication with each actuator (140), wherein the controller (610) is configured to respond to a plurality of different voice commands regarding flexion and extension of the joint (20), wherein each of the plurality of voice commands corresponds to a different predetermined movement pattern for the fingers and wherein the different predetermined movement patterns include: gripping an object (40), pinching the object (40), grasping the object (40) and releasing the object (40).

5. The exoskeleton system according to claim 1, further having a palm (30).

6. The exoskeleton system of Claim 5, further comprising:
(a) a plurality of suction cups (1010) disposed adjacent to the palm (30) so as to increase a gripping force applied to an object (40) being gripped;
(b) a suction cup controller; and
(c) a pneumatic tube (1022) coupling each suction cup (1010) to the suction cup controller so that the suction cup controller maintains a desired pressure with the suction cup (1010).

7. The exoskeleton system of Claim 6, further comprising a sensor (1011) embedded in at least one of the plurality of suction cups (1010) and configured to provide the suction cup controller with real time feedback.

## Patentansprüche

1. Exoskelett-System, das für den Einsatz mit einer Mehrzahl verschiedener Finger einer Hand ausgestaltet ist und eine Mehrzahl von Gelenkbewegungseinrichtungen (100) umfasst,
wobei die Gelenkbewegungseinrichtung (100) dazu ausgebildet ist, die selektive Beugung und Streckung eines Gelenks (20) durchzuführen, das ein erstes Gelenkelement (10) mit einem proximalen Ende (11) und einem gegenüberliegenden distalen Ende (13) sowie ein zweites Gelenkelement (12) mit einem proximalen Ende (15) und einem gegenüberliegenden distalen Ende (17) aufweist, und wobei das Gelenk das distale Ende (13) des ersten Gelenkelements (10) mit dem proximalen Ende (15) des zweiten Gelenkelements (12) koppelt, so dass das zweite Gelenkelement (12) in einer Beugungsbeziehung zum ersten Gelenkelement (10) in einer Biegestellung steht,
wobei jede der Mehrzahl der Gelenkbewegungseinrichtungen (100) an einem anderen zugeordneten Finger der Hand befestigt ist, so dass jede Gelenkbewegungseinrichtung (100) so ausgebildet ist, dass sie den zugeordneten Finger dazu veranlasst, sich auf vorbestimmte Weise zu bewegen,
wobei die Gelenkbewegungseinrichtung (100) umfasst:
(a) eine Sehne (120) mit einem ersten Ende und einem gegenüberliegenden zweiten Ende, die benachbart zu einer ausgewählten Seite des ersten Gelenkelements (10) und des zweiten Gelenkelements (12) angeordnet ist;
(b) eine Sehnenbefestigungseinrichtung (112), die am distalen Ende (17) des zweiten Gelenkelements (12) befestigt ist, an dem die Sehne (120) befestigt ist;
(c) mindestens einen Phalangenring (110), der am ersten Gelenkelement (10) befestigt ist, wobei der zumindest eine Phalangenring (110) und die Sehnenbefestigungseinrichtung (112) einen Sehnenführungsmechanismus (113) umfassen, der so ausgestaltet ist, dass er die Sehne (120) von der Sehnenbefestigungseinrichtung (112) durch den zumindest einen Phalangenring (110) führt;
(d) ein Aktor (140), der in einer festen Beziehung zum ersten Gelenkelement (10) steht und mit der Sehne (120) gekoppelt ist, wobei der Aktor (140) so ausgestaltet ist, dass er die Sehne (120) selektiv zu sich hinzieht und dadurch bewirkt, dass sich das Gelenk (20) beugt; und
(e) ein elastisches Element (130), das sowohl mit dem Phalangenring (110) als auch mit der Sehnenbefestigungseinrichtung (112) gekoppelt ist und so ausgestaltet ist, dass es eine Streckkraft auf die Sehnenbefestigungseinrichtung (112) ausübt, wodurch das Gelenk (20) gestreckt wird, wenn der Aktor (140) die Sehne freigibt; wobei der Aktor (140) umfasst:
(f) einen Sehnenverkürzungsmechanismus, der so ausgebildet ist, dass er einen Bereich der ersten Sehne (120) zwischen dem Phalangenring (110) und der Sehnenbefestigungsrichtung (112) verkürzt; und
(g) eine Steuereinrichtung (610), die so ausgebildet ist, dass sie den Sehnenverkürzungsmechanismus aktiviert, wobei der Sehnenverkürzungsmechanismus umfasst:
(h) ein Gehäuse (210);
(i) einen Elektromotor (212), der mit dem Gehäuse (210) gekoppelt ist;
(j) ein Gewindeelement (214), das mit dem Motor (212) gekoppelt ist, so dass es durch diesen gedreht werden kann; und
(k) eine Mutter (218), die in Gewindeverbindung mit dem Gewindeelement (214) steht, wobei die Mutter (218) vom Gehäuse (210) so gehalten wird, dass sich die Mutter (218) seitlich entlang des Gewindeelements (214) bewegt, wenn der Motor (212) das Gewindeelement (214) dreht, wobei die Mutter (218) einen Durchgang (218) definiert, durch den die Sehne (120) verläuft,
**dadurch gekennzeichnet, dass**
(l) das Gewindeelement (214) eine durchgehende Spiralwurzel (216) mit einer Steigung umfasst, die dem Durchmesser der Sehne (120) entspricht ist, wobei das zweite Ende der Sehne (120) am Gewindeelement (214) befestigt ist, so dass, wenn sich das Gewindeelement (214) in eine erste Richtung dreht, die Sehne (120) in das Gehäuse (210) gezogen und um die durchgehende Spiralwurzel (216) gewickelt wird, und dass, wenn sich das Gewindeelement (214) in eine zweite Richtung, entgegengesetzt zur ersten Richtung, dreht, die Sehne (120) von der durchgehenden Spiralwurzel (216) abgewickelt und aus dem Gehäuse (210) freigegeben wird.

2. Exoskelett-System nach Anspruch 1, wobei der Sehnenführungsmechanismus (113) jedes Phalangenrings (110) einen Durchgang definiert, durch den die Sehne (120) verläuft und der mit einer Poly(tetrafluorethylen)-Auskleidung ausgekleidet ist, um die Reibung an der Sehne (120) zu verringern.

3. Exoskelett-System, wobei das elastische Element (130) eine Feder (510) umfasst, deren eines Ende in fester Beziehung zum ersten Gelenk (20) steht und deren zweites Ende mit einer Schnur (520) gekoppelt ist, die an der Kappe(114) befestigt ist und auf einer der Sehne (120) gegenüberliegenden Seite des Gelenks (20) angeordnet ist.

4. Das Exoskelett-System nach Anspruch 1, wobei die Steuereinrichtung mit jedem Aktor (140) in Steuerungskommunikation steht, wobei die Steuereinrichtung (610) so ausgestaltet ist, dass sie auf eine Mehrzahl verschiedener Sprachbefehle bezüglich der Beugung und Streckung des Gelenks (20) reagiert, wobei jeder der Mehrzahl Sprachbefehle einem anderen vorbestimmten Bewegungsmuster für die Finger zugeordnet ist und wobei die verschiedenen vorbestimmten Bewegungsmuster Folgendes umfassen: das Greifen eines Objekts (40), das Einklemmen des Objekts (40), das Festhalten des Objekts (40) und das Loslassen des Objekts (40).

5. Exoskelett-System nach Anspruch 1, das ferner eine Handfläche (30) aufweist.

6. Exoskelett-System nach Anspruch 5, das ferner umfasst:
(a) eine Mehrzahl von Saugnäpfen (1010), die benachbart zur Handfläche (30) angeordnet sind, um eine auf einen zu greifenden Gegenstand (40) ausgeübte Greifkraft zu erhöhen;
(b) eine Saugnapf-Steuereinrichtung; und
(c) eine pneumatische Leitung (1022), die jeden Saugnapf (1010) mit der Saugnapf-Steuereinrichtung koppelt, so dass die Saugnapf-Steuereinrichtung einen gewünschten Druck im Saugnapf (1010) aufrechterhält.

7. Exoskelett-System nach Anspruch 6, das ferner einen Sensor (1011) umfasst, der in mindestens einen der mehreren Saugnäpfe (1010) eingebettet und so ausgestaltet ist, dass er der Saugnapf-Steuereinrichtung Echtzeit-Rückmeldung bereitstellt.

## Revendications

1. Système d'exosquelette configuré pour être utilisé en lien avec une pluralité de doigts différents d'une main et comprenant une pluralité de dispositifs de mobilisation des articulations (100),
dans lequel :
le dispositif de mobilisation des articulations (100) est adapté pour la flexion et l'extension sélectives d'une articulation (20) qui comporte un premier élément d'articulation (10) qui comporte une extrémité proximale (11) et une extrémité distale opposée (13) et un second élément d'articulation (12) qui comporte une extrémité proximale (15) et une extrémité distale opposée (17), et pour le couplage articulaire de l'extrémité distale (13) du premier élément d'articulation (10) avec l'extrémité proximale (15) du second élément d'articulation (12) de telle sorte que le second élément d'articulation (12) soit selon une relation de flexion par rapport au premier élément d'articulation (10),
dans lequel :
chacun de la pluralité de dispositifs de mobilisation des articulations (100) est fixé sur un doigt correspondant différent de la main de telle sorte que chaque dispositif de mobilisation des articulations (100) soit configuré pour forcer le doigt correspondant à se déplacer d'une manière prédéterminée,
dans lequel :
le dispositif de mobilisation des articulations (100) comprend :
(a) un élément de précontrainte (120) qui comporte une première extrémité et une seconde extrémité opposée et qui est disposé de manière à être adjacent à un côté sélectionné du premier élément d'articulation (10) et du second élément d'articulation (12) ;
(b) un dispositif de fixation d'élément de précontrainte (112) qui est fixé à l'extrémité distale (17) du second élément d'articulation (12) auquel l'élément de précontrainte (120) est fixé ;
(c) au moins un anneau phalangien (110) qui est fixé au premier élément d'articulation (10), l'au moins un anneau phalangien (110) et le dispositif de fixation d'élément de précontrainte (112) incluant un mécanisme de guidage d'élément de précontrainte (113) qui est configuré pour guider l'élément de précontrainte (120) depuis le dispositif de fixation d'élément de précontrainte (112) au travers de l'au moins un anneau phalangien (110) ;
(d) un actionneur (140) qui est selon une relation fixée avec le premier élément d'articulation (10) et qui est couplé avec l'élément de précontrainte (120), l'actionneur (140) étant configuré pour, de façon sélective, tirer l'élément de précontrainte (120) vers l'intérieur, d'où ainsi l'obtention de la flexion de l'articulation (20) ; et
(e) un élément élastique (130) qui est couplé à la fois avec l'anneau phalangien (110) et avec le dispositif de fixation d'élément de précontrainte (112) et qui est configuré pour appliquer une force d'extension sur le dispositif de fixation d'élément de précontrainte (112), d'où ainsi l'obtention de l'extension de l'articulation (20) lorsque l'actionneur (140) libère l'élément de précontrainte,
dans lequel :
l'actionneur (140) comprend :
(f) un mécanisme de raccourcissement d'élément de précontrainte qui est configuré pour raccourcir une partie du premier élément de précontrainte (120) entre l'anneau phalangien (110) et le dispositif de fixation d'élément de précontrainte (112) ; et
(g) un contrôleur (610) qui est configuré pour activer le mécanisme de raccourcissement d'élément de précontrainte ;
dans lequel :
le mécanisme de raccourcissement d'élément de précontrainte comprend :
(h) un boîtier (210) ;
(i) un moteur électrique (212) qui est couplé avec le boîtier (210) ;
(j) un élément fileté (214) qui est couplé avec le moteur (212) de manière à pouvoir être ainsi entraîné en rotation ; et
(k) un écrou (218) qui est selon une coopération filetée avec l'élément fileté (214), l'écrou (218) étant maintenu par le boîtier (210) de telle sorte que lorsque le moteur (212) entraîne en rotation l'élément fileté (214), l'écrou (218) soit déplacé latéralement le long de l'élément fileté (214), l'écrou (218) définissant un passage (218) au travers duquel l'élément de précontrainte (120) passe ;
**caractérisé en ce que** :
l'élément fileté (214) inclut une racine en spirale continue (216) qui présente un pas qui correspond à un diamètre de l'élément de précontrainte (120), dans lequel la seconde extrémité de l'élément de précontrainte (120) est fixée à l'élément fileté (214) de telle sorte que lorsque l'élément fileté (214) est entraîné en rotation dans une première direction, l'élément de précontrainte (120) soit tiré à l'intérieur du boîtier (210) et soit enroulé autour de la racine en spirale continue (216) et de telle sorte que lorsque l'élément fileté (214) est entraîné en rotation dans une seconde direction, opposée à la première direction, l'élément de précontrainte (120) soit déroulé à partir de la racine en spirale continue (216) et soit libéré du boîtier (210).

2. Système d'exosquelette selon la revendication 1, dans lequel le mécanisme de guidage d'élément de précontrainte (113) de chaque anneau phalangien (110) inclut un passage au travers duquel l'élément de précontrainte (120) passe et qui est défini de telle sorte qu'il soit chemisé au moyen d'un revêtement à base de polytétrafluoroéthylène de manière à réduire la friction sur l'élément de précontrainte (120).

3. Système d'exosquelette selon la revendication 1, dans lequel l'élément élastique (130) comprend un ressort (510) qui comporte une première extrémité qui est selon une relation fixée avec la première articulation (20) et une seconde extrémité qui est couplée avec un cordon (520) qui est couplé avec un capuchon (114) et qui est disposé sur un côté de l'articulation (20) opposé à l'élément de précontrainte (120).

4. Système d'exosquelette selon la revendication 1, dans lequel le contrôleur est en communication en termes de commande(s) avec chaque actionneur (140), dans lequel le contrôleur (610) est configuré pour répondre à une pluralité de commandes vocales différentes qui concernent la flexion et l'extension de l'articulation (20), dans lequel chacune de la pluralité de commandes vocales correspond à une configuration de mobilisation prédéterminée différente pour les doigts et dans lequel les configurations de mobilisation prédéterminées différentes incluent : la prise en main d'un objet (40), l'application d'une force de serrage sur l'objet (40), la saisie de l'objet (40) et la libération de l'objet (40).

5. Système d'exosquelette selon la revendication 1, comportant en outre une paume (30).

6. Système d'exosquelette selon la revendication 5, comprenant en outre :
(a) une pluralité d'interfaces à fonction de ventouse (1010) qui sont disposées de telle sorte qu'elles soient adjacentes à la paume (30) de manière à augmenter une force de prise en main qui est appliquée sur un objet (40) qui est en cours de prise en main ;
(b) un contrôleur d'interface(s) à fonction de ventouse ; et
(c) un tube pneumatique (1022) qui couple chaque interface à fonction de ventouse (1010) avec le contrôleur d'interface(s) à fonction de ventouse de telle sorte que le contrôleur d'interface(s) à fonction de ventouse maintienne une pression souhaitée en lien avec l'interface à fonction de ventouse (1010).

7. Système d'exosquelette selon la revendication 6, comprenant en outre un capteur (1011) qui est intégré dans au moins l'une de la pluralité d'interfaces à fonction de ventouse (1010) et qui est configuré pour fournir un retour en temps réel au contrôleur d'interface(s) à fonction de ventouse.
